# EUROPEAN PATENT APPLICATION

(11) **EP 2 922 018 A1**
(43) Date of publication of application: **23.09.2015**
(21) Application number: 12888222.2
(22) Date of filing: 14.11.2012
(51) Int. Cl.: G06Q 50/24

(54) **MEDICAL INFORMATION ANALYSIS PROGRAM, MEDICAL INFORMATION ANALYSIS DEVICE, AND MEDICAL INFORMATION ANALYSIS METHOD**

(71) Applicant: Fujitsu Limited, Kawasaki-shi, Kanagawa 211-8588 (JP)
(72) Inventor: HAYAKAWA, Tatsuo, Osaka-shi Osaka 540-8514 (JP)
(74) Representative: Hoffmann Eitle
(86) International application number: PCT/JP2012/079498
(87) International publication number: WO 2014/076777

(57) **Abstract**

It becomes possible to extract information useful for the medical care of a certain patient from medical records.

A medical information analysis apparatus (S) includes a specifying means (2) and an extraction means (4). The specifying means (2) specifies a second patient whose medical record is similar to that of a first patient with reference to the medical records of a plurality of patients. The extraction means (4) extracts information from the medical record of the second patient.

## Description

### Technical Field

The embodiments discussed herein relate to a medical information analysis program, medical information analysis apparatus, and medical information analysis method for analyzing medical information.

### Background Art

With advances in information technology, the range of computer use has expanded. As a result, computers store an enormous amount of electronic data. A lot of the electronic data includes character information. Therefore, some techniques have been considered to efficiently use such character information included in electronic data. For example, for Q&A service on the Internet, there is a technique for providing users, if they have posted any questions or answers even without clearly representing or answering about their interests, with recommended questions according to their knowledge and interests to motivate them to answer. In addition, there is a method for collecting, managing, searching, and sharing personal information manually entered as comments.

Data management using computers has become widespread also in the healthcare industry. For example, in the healthcare industry, electronic medical charts are increasingly adopted mainly by large-scale hospitals in order to manage diagnosis results and other reports for each patient. In the field of healthcare, it has been considered to efficiently use accumulated data on a large number of patients for medical care. For example, in systems for searching for similar images on the basis of text added to images, there is a system for finding desired images promptly and easily. This system makes it possible to automatically extract, with respect to an image, keywords or feature values that a searcher might overlook or might not be aware of and also to find related cases that a doctor might overlook. In addition, there is a medical diagnosis report system for preventing radiologists from overlooking observation points and thereby for providing high-quality radiological reports. This medical diagnosis report system extracts terms for specifying radiologic interpretation points on the basis of observations written on medical diagnosis reports as check items for radiologic interpretation, obtains sentences including the extracted radiologic interpretation check items from observations written on a medical diagnosis report, and displays the sentences for each of the radiologic interpretation check items.

### Citation List

### Patent Literature

PTL1: Japanese Laid-open Patent Publication No. 2011-186854
PTL2: Japanese Laid-open Patent Publication No. 2005-85285 PTL3: Japanese Laid-open Patent Publication No. 2004-157623
PTL4: Japanese Laid-open Patent Publication No. 2011-100254

### Summary of Invention

### Technical Problem

Various methods have been proposed for use of medical information. However, conventional techniques are not yet sufficient to make good use of a massive amount of data on medical care. For example, for the medical care of a patient, the medical records (charts) of other patients suffering from the same symptoms as the patient under medical care are considered useful. However, in the current situation, it is not possible to appropriately determine which of a great number of medical records contains information that is worth considering for the medical care of a patient. That is to say, it is not possible to extract information useful for the patient from the great number of accumulated medical records.

According to one aspect, the present invention is intended to provide a medical information analysis program, medical information analysis apparatus, and medical information analysis method for extracting information useful for the medical care of a patient from medical records.

### Solution to Problem

According to one aspect, there is provided a medical information analysis program that causes a computer to perform a process including: specifying a second patient whose medical record is similar to that of a first patient with reference to the medical records of a plurality of patients, and extracting information from the medical record of the second patient.

### Advantageous Effects of Invention

According to one aspect, it becomes possible to extract information useful for the medical care of a patient from medical records.

The above and other objects, features, and advantages of the invention will become more apparent from the following detailed description taken in conjunction with the following drawings illustrating a preferred embodiment of the present invention by way of example.

### Brief Description of Drawings

[FIG. 1] FIG. 1 illustrates an example of a functional configuration of a medical information analysis apparatus according to a first embodiment.
[FIG. 2] FIG. 2 illustrates an example of a system configuration according to a second embodiment.
[FIG. 3] FIG. 3 illustrates an example of a hardware configuration of a server that is employed in the embodiment.
[FIG. 4] FIG. 4 is a block diagram illustrating a recommendation function of the server.
[FIG. 5] FIG. 5 illustrates an example of a data structure of an electronic medical chart storage unit.
[FIG. 6] FIG. 6 illustrates an example of a data structure of a registration exclusion dictionary storage unit.
[FIG. 7] FIG. 7 is a flowchart illustrating an example of how to perform a recommendation process.
[FIG. 8] FIG. 8 is a flowchart illustrating an example of how to perform a target-patient record analysis process.
[FIG. 9] FIG. 9 illustrates an example of a data structure of a target-patient-record-based term storage unit.
[FIG. 10] FIG. 10 is a flowchart illustrating an example of how to perform an other-patient record analysis process.
[FIG. 11] FIG. 11 illustrates an example of a data structure of a similar-record-based term storage unit.
[FIG. 12] FIG. 12 illustrates similarity relationships between record notes.
[FIG. 13] FIG. 13 is a flowchart illustrating how to perform a similar patient determination process.
[FIG. 14] FIG. 14 illustrates an example of a data structure of a similar patient information storage unit.
[FIG. 15] FIG. 15 illustrates an example of a data structure of a similar-patient-record-based term storage unit.
[FIG. 16] FIG. 16 is a flowchart illustrating how to perform a reference term extraction process.
[FIG. 17] FIG. 17 illustrates an example of recommendation.

### Description of Embodiments

Hereinafter, embodiments will be described with reference to the accompanying drawings. Features of the embodiments may be combined unless they exclude each other.

### [First Embodiment]

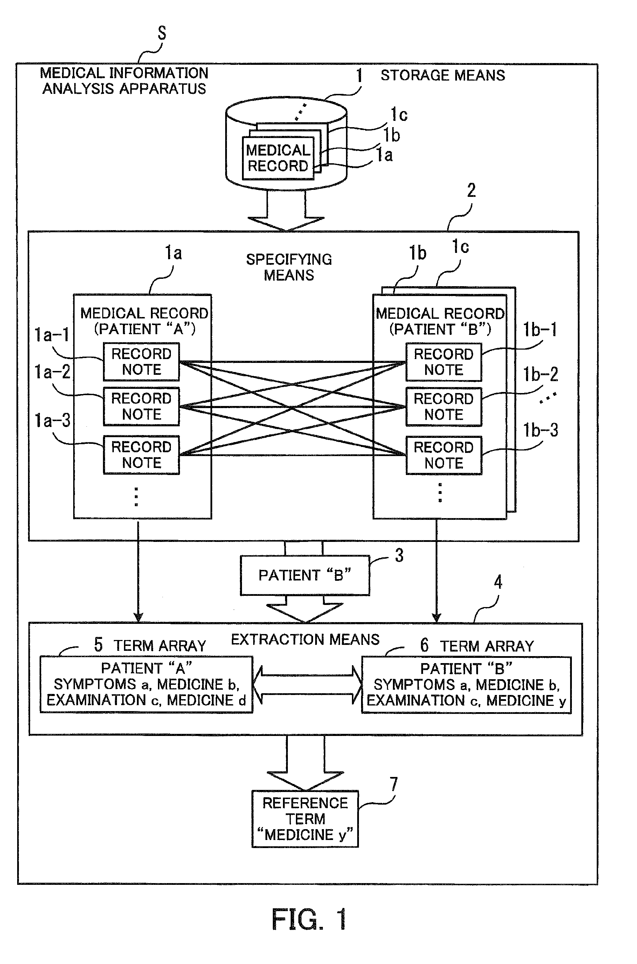

FIG. 1 illustrates an example of a functional configuration of a medical information analysis apparatus according to a first embodiment. A medical information analysis apparatus S includes a storage means 1, a specifying means 2, and an extraction means 4. For example, the medical information analysis apparatus S is a computer that runs a medical information analysis program to analyze medical information.

The storage means 1 stores the medical records (charts) 1a, 1b, 1c, ... of a plurality of patients. These medical records 1a, 1b, and 1c, ... are created for the individual patients. The medical records 1a, 1b, and 1c, ... include record notes indicating the details of the medical care and nursing care for the patients.

The specifying means 2 specifies a second patient whose medical record is similar to that of a first patient with reference to the medical records of the plurality of patients. For example, the specifying means 2 calculates the degree of similarity between each of the plurality of record notes 1a-1, 1a-2, 1a-3, ... included in the medical record la of the first patient and each of the plurality of record notes 1b-1, 1b-2, 1b-3, ... included in the medical records 1b, 1c, ... of the other patients. The specifying means 2 then calculates the total degree of similarity between record notes for each of the other patients. A total degree of similarity represents how similar one of the other patients is to the first patient. Alternatively, the specifying means 2 may be designed to extract a predetermined number of record notes of the other patients in descending order of the degree of similarity, with respect to each of the plurality of record notes 1a-1, 1a-2, 1a-3, ... included in the medical record la of the first patient, and calculate the total degree of similarity of the extracted record notes for each of the other patients.

The extraction means 4 extracts information from the medical record 1b of the second patient. For example, the extraction means 4 extracts terms that do not occur in the medical record 1a of the first patient from the terms occurring in the medical record 1b of the second patient.

In the above-described medical information analysis apparatus S, analysis of medical information is started in response to an analysis instruction specifying a first patient from, for example, a user. In the analysis of medical information, the specifying means 2 first calculates the degree of similarity between the medical record 1a of the first patient and each of the medical records 1b, 1c, ... of the other patients. For example, the specifying means 2 calculates the degree of similarity between each of 1a-1, 1a-2, 1a-3, ... included in the medical record 1a of the first patient and each of the plurality of record notes 1b-1, 1b-2, 1b-3, ... included in the medical records 1b, 1c, ... of the other patients. The specifying means 2 then calculates the total degree of similarity between record notes for each of the other patients. Then, the specifying means 2 specifies one or more other patients, in descending order of the total degree of similarity, as second patients. When specifying the second patients, the specifying means 2 sends, for example, information (similar patient information 3) indicating the specified patients to the extraction means 4. Referring to the example of FIG. 1, a patient "B" is specified as a second patient.

Then, the extraction means 4 extracts terms that are considered useful for the medical care of the first patient, from the medical record 1b of the second patient. For example, the extraction means 4 extracts terms (term array 5) that do not occur in the medical record 1a of the first patient from the terms (term array 6) occurring in the medical record 1b of the second patient. The extraction means 4 then outputs the extracted terms, for example, as terms (reference terms 7) that are useful for the medical care of the first patient 1.

As described above, information that is useful for the medical care of the first patient is extracted from the medical record of the second patient which has similar medical data to that of the first patient, thereby promoting effective use of medical information. This results in improving the quality of medical care.

In this connection, the number of second patients specified by the specifying means 2 is not limited to one. For example, the specifying means 2 may be designed to calculate the degree of similarity between the medical record of the first patient and each of the medical records of the other patients, and specify a predetermined number of other patients in descending order of the degree of similarity as second patients.

In addition, in calculating the degree of similarity between record notes, the specifying means 2 may perform weighting based on the types of the record notes. For example, the specifying means 2 multiplies a result of similarity calculation based on the contents of two record notes, which are objects for calculating the degree of similarity, by the weights corresponding to the two record notes, and takes the multiplication result as the degree of similarity between the two record notes. This makes it possible to give importance to record notes according to their types and extract appropriate terms. For example, a weight for medical data recorded by doctors is set higher than that for medical data recorded by nurses, so as to preferentially specify other patients who have similar details of medical care, rather than the details of nursing care, as second patients.

In addition, physical information indicating physical features of patients may be included in the medical records of the patients. By determining the degree of similarity between medical records using such physical information, other patients who have similar physical information to the first patient are specified as second patients. This makes it possible to extract terms that are useful for treating a disease whose treatment is greatly different depending on differences in the physical information of patients.

In this connection, the specifying means 2 and extraction means 4 are implemented by, for example, using a processor provided in the medical information analysis apparatus S. In addition, the storage means 1 may be implemented by, for example, using a RAM (Random Access Memory) provided in the medical information analysis apparatus S.

### [Second Embodiment]

The following describes a second embodiment. The second embodiment relates to an electronic medical chart management system which allows a plurality of doctors to share knowledge recorded in electronic medical charts over a network within a hospital.

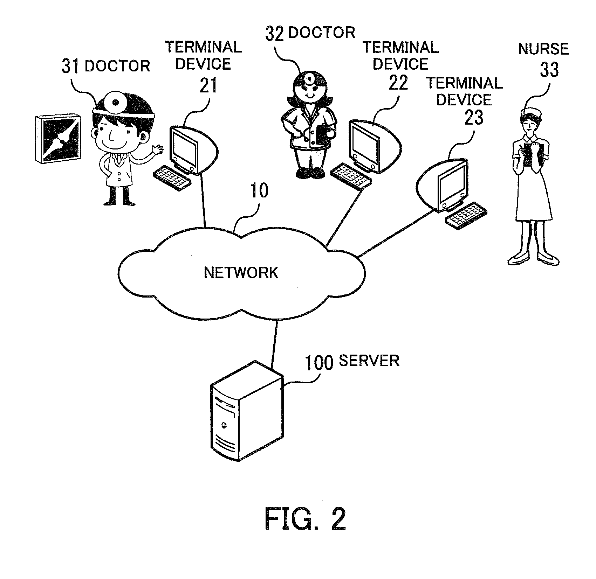

FIG. 2 illustrates an example of a system configuration according to the second embodiment. In an electronic medical chart management system, a server 100 and a plurality of terminal devices 21 to 23 are connected over a network 10. The terminal devices 21 to 23 are used by doctors 31 and 32 and a nurse 33 to enter information about the medical care of patients. The information entered in the terminal devices 21 to 23 are sent to the server 100 over the network 10 and are recorded in the server 100. A collection of record notes for each patient is treated as an electronic medical chart for the patient. Each record note is information that is recorded each time a treatment or nursing care is provided.

The server 100 is a computer that manages electronic medical charts. The server 100 analyzes accumulated electronic medical charts in response to a recommendation request from a terminal device 21 to 23, and outputs a recommendation result. The recommendation request specifies a patient (target patient) for recommendation. For example, the server 100 extracts keywords that are not recorded in the electronic medical chart of the target patient but are recorded in the electronic medical charts of other patients (similar patients) whose symptoms or treatment histories are similar to those of the target patient, and outputs the keywords as a recommendation result.

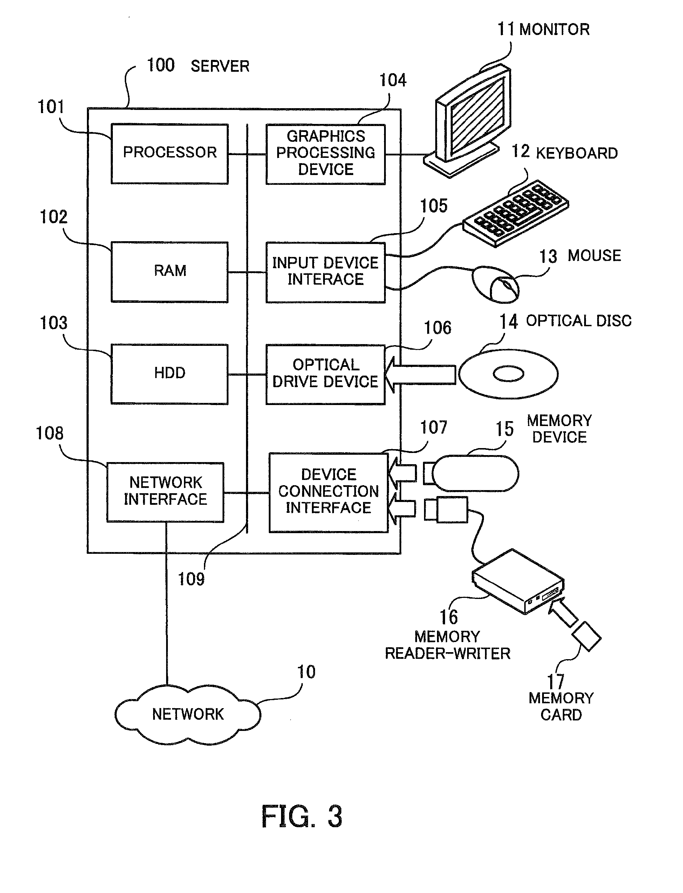

FIG. 3 illustrates an example of a hardware configuration of a server that is employed in the embodiment. The server 100 is entirely controlled by a processor 101. A RAM 102 and a plurality of peripherals are connected to the processor 101 via a bus 109. The processor 101 may be a multiprocessor. The processor 101 may be, for example, a CPU (Central Processing Unit), a MPU (Micro Processing Unit), or a DSP (Digital Signal Processor). Some or all of the functions of the processor 101 may be implemented by using an ASIC (Application Specific Integrated Circuit), a PLD (Programmable Logic Device), or other electronic circuits.

The RAM 102 is used as a primary storage device of the server 100. The RAM 102 temporarily stores at least part of OS (Operating System) programs and application programs to be executed by the processor 101. The RAM 102 also stores various data to be used while the processor 101 operates.

The peripherals connected to the bus 109 include an HDD (Hard Disk Drive) 103, a graphics processing device 104, an input device interface 105, an optical drive device 106, a device connection interface 107, and a network interface 108.

The HDD 103 magnetically writes and reads data on a built-in disk. The HDD 103 is used as a secondary storage device of the server 100. The HDD 103 stores the OS programs, application programs, and various data. As a secondary storage device, a semiconductor storage device, such as a flash memory, may be used.

A monitor 11 is connected to the graphics processing device 104. The graphics processing device 104 displays images on the screen of the monitor 11 in accordance with instructions from the processor 101. As the monitor 11, a display device using CRT (Cathode Ray Tube), a liquid crystal display device, or the like may be used.

A keyboard 12 and a mouse 13 are connected to the input device interface 105. The input device interface 105 gives the processor 101 signals received from the keyboard 12 and mouse 13. The mouse 13 is one example of pointing devices, and another pointing device may be used. Other pointing devices include, for example, a touch panel, a tablet, a touchpad, a track ball, and so on.

The optical drive device 106 reads data from an optical disc 14 with laser light or the like. The optical disc 14 is a portable recording medium on which data is recorded so as to be read with reflection of light. As the optical disc 14, a DVD (Digital Versatile Disc), DVD-RAM, CD-ROM (Compact Disc Read Only Memory), CD-R (Readable), CD-RW (ReWritable), or the like may be used.

The device connection interface 107 is a communication interface that allows peripherals to be connected to the server 100. For example, a memory device 15 and a memory reader-writer 16 may be connected to the device connection interface 107. The memory device 15 is a recording medium provided with a function for communication with the device connection interface 107. The memory reader-writer 16 is a device that performs data read and write on a memory card 17. The memory card 17 is a card-type recording medium.

The network interface 108 is connected to the network 10. The network interface 108 performs data communication with other computers or communication devices over the network 10.

With the above hardware configuration, the processing functions of the second embodiment may be implemented. In this connection, the medical information analysis apparatus of the first embodiment may be configured with the same hardware as the server 100 of FIG. 3.

The server 100 performs the processing functions of the second embodiment by, for example, executing a program recorded on a computer-readable recording medium. The program describing the contents of processing to be performed by the server 100 may be recorded on a variety of recording media. For example, the program to be executed by the server 100 may be stored on the HDD 103. The processor 101 loads at least part of the program from the HDD 103 to the RAM 102 and executes the program. Alternatively, the program to be executed by the server 100 may be stored on a portable recording medium, such as the optical disc 14, memory device 15, or memory card 17. The program stored in the portable recording medium is installed in the HDD 103, for example, under the control of the processor 101, and thereby becomes executable. The processor 101 may read and execute the program directly from the portable recording medium.

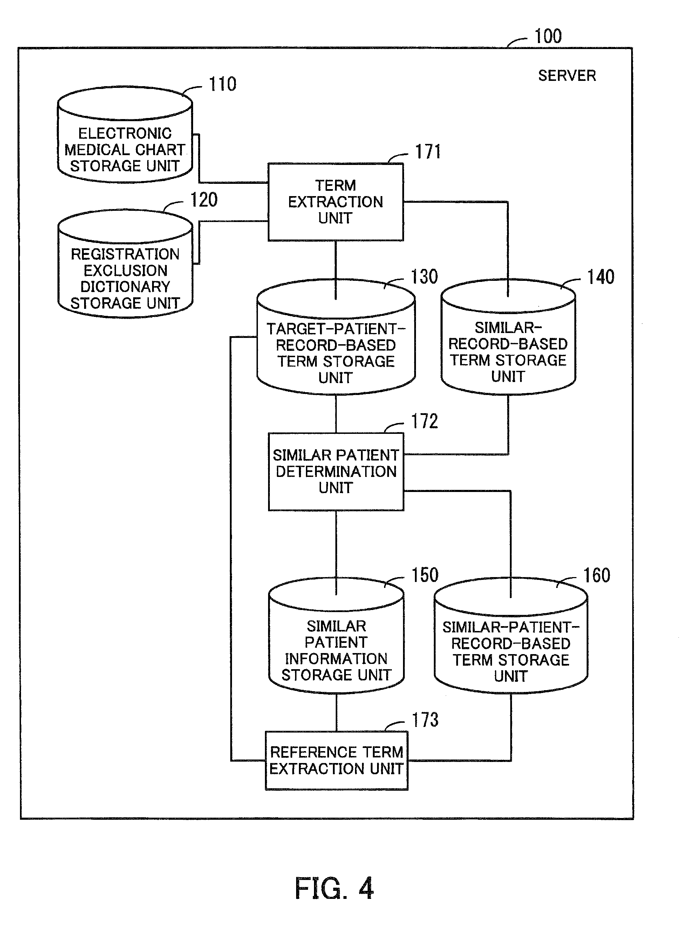

FIG. 4 is a block diagram illustrating a recommendation function of the server. The server 100 includes, as data storage units, an electronic medical chart storage unit 110, a registration exclusion dictionary storage unit 120, a target-patient-record-based term storage unit 130, a similar-record-based term storage unit 140, a similar patient information storage unit 150, and a similar-patient-record-based term storage unit 160. For example, part of the storage area of the RAM 102 or HDD 103 of the server 100 is used for these data storage units. The server 100 also includes, as data processing units, a term extraction unit 171, a similar patient determination unit 172, and a reference term extraction unit 173. These processing units are implemented by, for example, the processor 101 of the server 100 executing a program stored in the RAM 102 or HDD 103.

The electronic medical chart storage unit 110 stores record notes entered by the doctors 31 and 32, the nurse 33, and so on. The registration exclusion dictionary storage unit 120 stores terms that are to be excluded from being extracted in a process of extracting terms from electronic medical charts. The target-patient-record-based term storage unit 130 stores terms extracted from the record notes of a patient (target patient) who is a target for recommendation. The similar-record-based term storage unit 140 stores terms extracted from similar record notes to the record notes of the target patient. The similar patient information storage unit 150 stores information on other patients (similar patients) whose electronic medical charts have similar contents to that of the target patient. The similar-patient-record-based term storage unit 160 stores terms included in the record notes of the similar patients.

When receiving a recommendation request specifying a target patient, the term extraction unit 171 obtains the record notes included in the electronic medical chart of the target patient from the electronic medical chart storage unit 110. The term extraction unit 171 then extracts terms from the obtained record notes. For example, the term extraction unit 171 performs morphological analysis to obtain a plurality of terms from the record notes. Then, the term extraction unit 171 eliminates terms included in a registration exclusion dictionary stored in the registration exclusion dictionary storage unit 120 from the terms obtained through the morphological analysis. In this connection, the term extraction unit 171 may refer to, for example, a medical term dictionary to eliminate terms that are not included in the medical term dictionary. The term extraction unit 171 stores the terms remaining after the elimination of such terms, in the target-patient-record-based term storage unit 130.

In addition, the term extraction unit 171 extracts terms from record notes that are similar to any of the record notes of the target patient, among the record notes of the other patients than the target patient. For example, the term extraction unit 171 performs morphological analysis on the record notes of the other patients to obtain a plurality of terms therefrom. The term extraction unit 171 then refers to the registration exclusion dictionary storage unit 120 to eliminate terms included in the registration exclusion dictionary from the terms obtained through the morphological analysis. Then, the term extraction unit 171 stores the terms remaining after the elimination of such terms in the similar-record-based term storage unit 140.

The similar patient determination unit 172 ranks the other patients according to the degrees of similarity in record notes between the target patient and the other patients. The similar patient determination unit 172 stores information on a predetermined number of patients (similar patients), in descending order of the degree of similarity, in the similar patient information storage unit 150. In addition, the similar patient determination unit 172 stores the terms extracted from the record notes of the similar patients in the similar-patient-record-based term storage unit 160.

The reference term extraction unit 173 extracts, as terms that are useful for the medical care of the target patient, the terms that are not included in the record notes of the target patient from the terms extracted from the record notes of the similar patients. The reference term extraction unit 173 then sends the extracted terms as a recommendation result to the terminal device having made the recommendation request. The terminal device displays the recommendation result.

Lines connecting between units illustrated in FIG. 4 represent part of communication paths, and communication paths other than the illustrated ones may be configured. Further, the similar patient determination unit 172 is one example of the specifying means 2 of the first embodiment illustrated in FIG. 1. Still further, the reference term extraction unit 173 is one example of the extraction means 4 of the first embodiment illustrated in FIG. 1.

Among the data storage units of the server 100, the electronic medical chart storage unit 110 and registration exclusion dictionary storage unit 120 have data registered by an administrator before the recommendation process is performed.

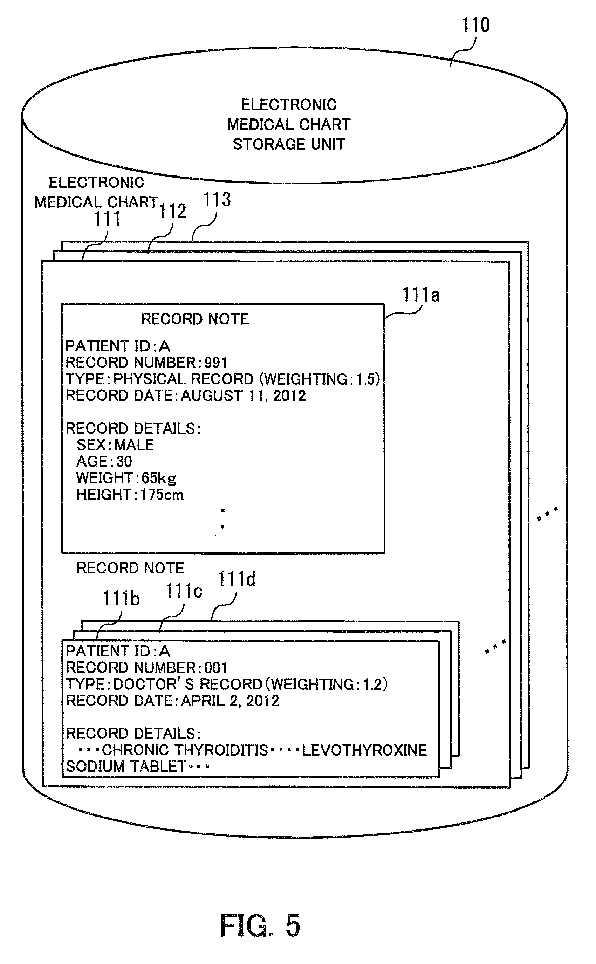

FIG. 5 illustrates an example of a data structure of an electronic medical chart storage unit. The electronic medical chart storage unit 110 stores electronic medical charts 111, 112, 113, ... for individual patients. The electronic medical chart 111 includes record notes 111a, 111b, 111c, 111d, .... Similarly to the electronic medical chart 111, the other electronic medical charts 112, 113, ... include record notes.

Each of the record notes 111a, 111b, 111c, 111d, ... includes patient ID, record number, type, record date, and record details. A patient ID is an identifier identifying a patient. A record number is an identifier identifying a record note within the server 100. A type indicates the type of a record note. For example, there are "physical record," "doctor's record", and "nurse's record" types. In this connection, a weight may be defined for each type of record notes. For example, a weight for doctor's records is set higher than that for nurse's records. For example, a result of similarity calculation between record notes is multiplied by their corresponding weight values. This evaluates a degree of similarity between doctor's records higher than that between nurse's records. A record date indicates when a record note was recorded.

In this connection, a weight for each type may previously be defined by a system administrator, for example. Alternatively, a user may define desired weights by specifying a weight for each type in a recommendation request.

The record note 111a of the "physical record" type includes the name and physical information of a patient. The physical information indicates physical features including sex, age, weight, height and so on. With regard to information which may vary with time, such as age, weight, or height, the latest information is set.

The record note 111b of the "doctor's record" type indicates the details of doctor's medical care. For example, the record note 111b indicates patient's symptoms, the names of prescribed medicines, and others.

Although not exemplified in FIG. 5, the "nurse's record" type of record notes indicate patients' conditions noticed during nursing care, for example.

Other types of record notes may include clinical records indicating the results of clinical care, rehabilitation records indicating the progresses of rehabilitation, and others, for example.

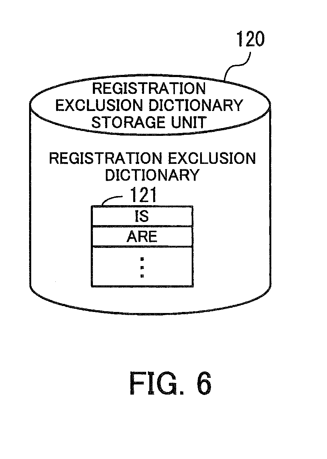

FIG. 6 illustrates an example of a data structure of a registration exclusion dictionary storage unit. The registration exclusion dictionary storage unit 120 stores a registration exclusion dictionary 121. The registration exclusion dictionary 121 contains terms that are to be excluded from being extracted from record notes. For example, terms, such as "is" or "are", are registered in the registration exclusion dictionary 121.

The server 100 performs the recommendation process using the electronic medical charts 111, 112, 113, ... and registration exclusion dictionary 121 illustrated in FIGS. 5 and 6.

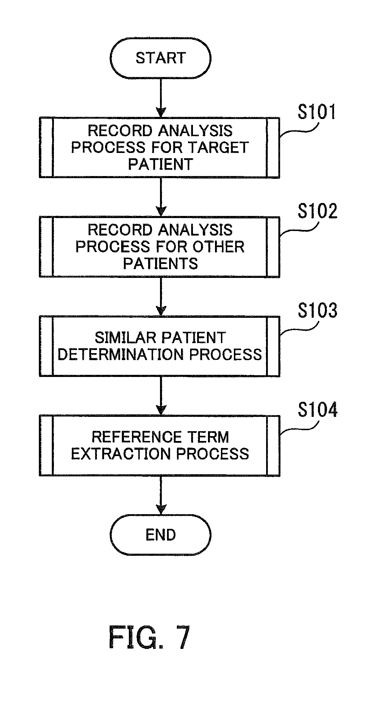

FIG. 7 is a flowchart illustrating an example of how to perform a recommendation process. In this connection, the recommendation process is performed when a recommendation request specifying a target patient is made from any terminal device.

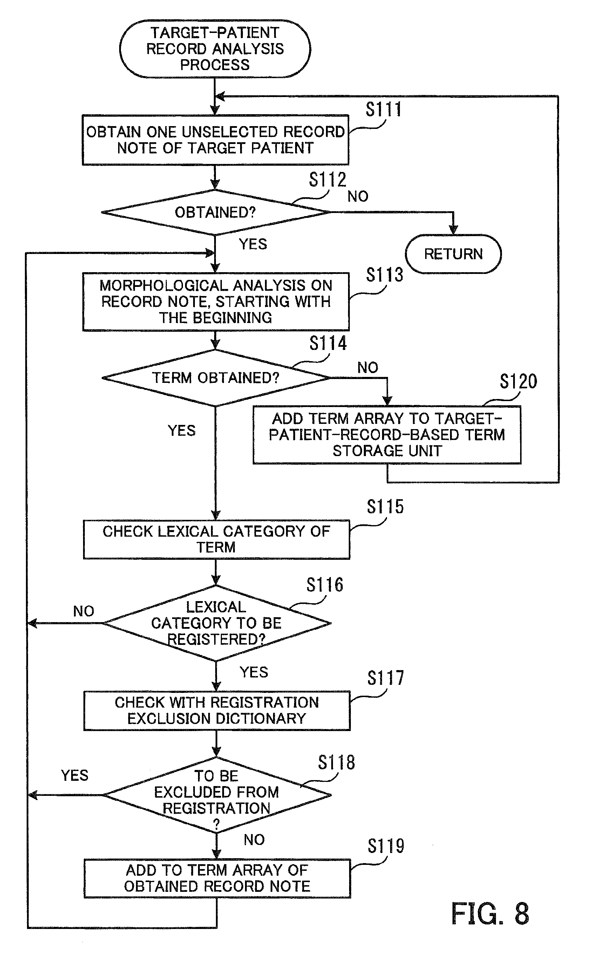

(Step S101) The term extraction unit 171 performs a record analysis process for the target patient. Through this process, the terms extracted from the record notes included in the electronic medical chart of the target patient are stored in the target-patient-record-based term storage unit 130. This target-patient record analysis process will be described in detail later (see FIG. 8).

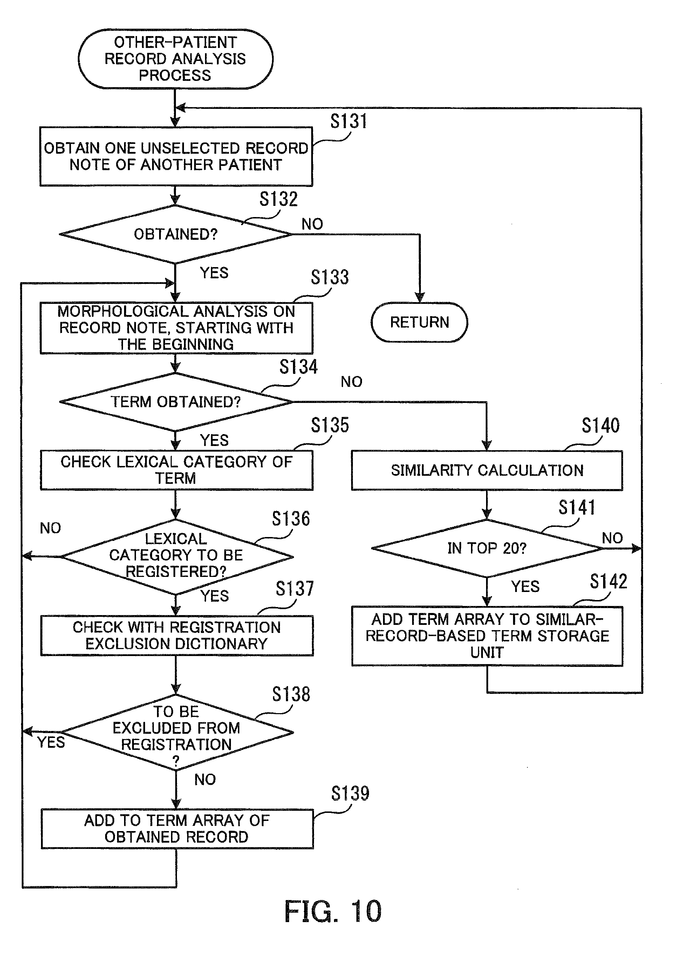

(Step S102) The term extraction unit 171 performs a record analysis process for the patients (other patients) other than the target patient. Through this process, the terms extracted from the record notes having similar contents to those of the electronic medical chart of the target patient among the record notes included in the electronic medical charts of the other patients are stored in the similar-record-based term storage unit 140. This other-patient record analysis process will be described in detail later (see FIG. 10).

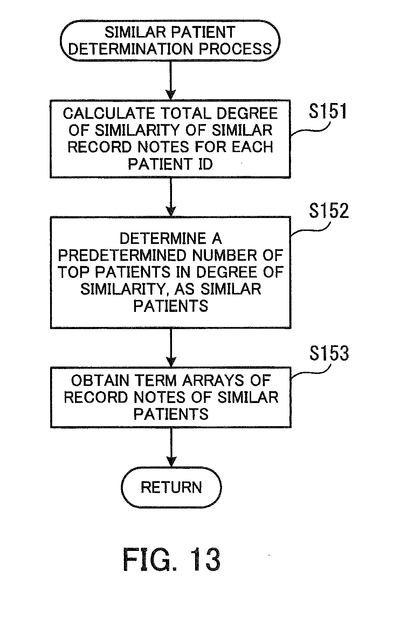

(Step S103) The similar patient determination unit 172 performs a similar patient determination process to find other patients (similar patients) similar to the target patient. Through this process, the patient IDs of a predetermined number of patients with high degrees of similarity are stored in the similar patient information storage unit 150. In addition, the terms extracted from the record notes of the electronic medical charts of the similar patients are stored in the similar-patient-record-based term storage unit 160. This similar patient determination process will be described in detail later (see FIG. 13).

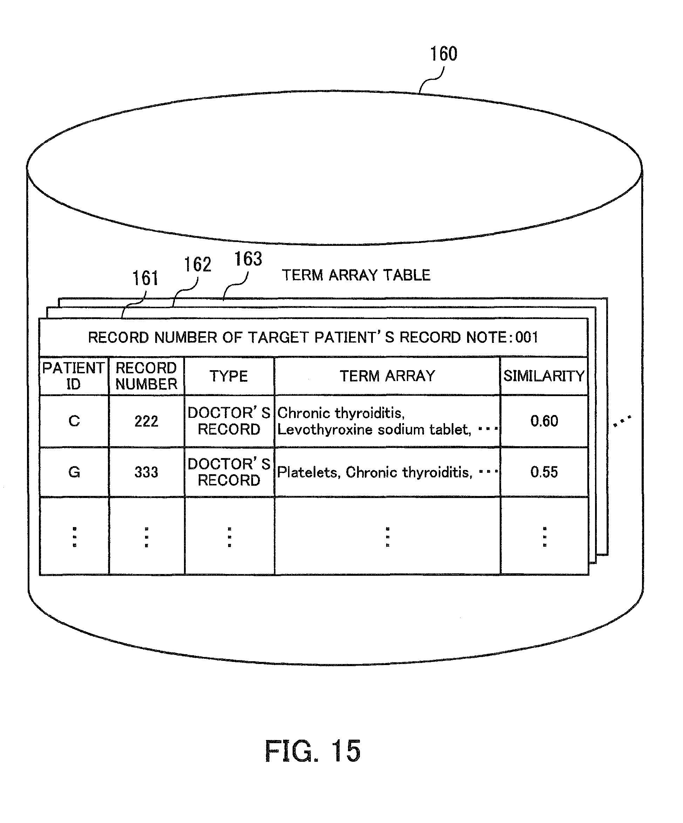

(Step S104) The reference term extraction unit 173 performs a reference term extraction process to extract terms (reference terms) that are useful for the medical care of the target patient. The extracted reference terms are sent to the terminal device having made the recommendation request. Then, the reference terms are displayed on the monitor of the terminal device. This reference term extraction process will be described in detail later (see FIG. 15).

The recommendation is performed in the way as described above. The following describes in detail each step of FIG. 7.

FIG. 8 is a flowchart illustrating an example of how to perform a target-patient record analysis process. The target-patient record analysis process is performed when a recommendation request is made.

(Step S111) The term extraction unit 171 obtains one unselected record note included in the electronic medical chart of the target patient from the electronic medical chart storage unit 110. In this connection, the term extraction unit 171 may previously filter the record notes of the electronic medical chart of the target patient with SQL (Structured Query Language). In this case, the term extraction unit 171 sequentially obtains the record notes remaining after the filtering.

(Step S112) The term extraction unit 171 determines whether any record note was obtained at step S111. If the electronic medical chart of the target patient includes one or more record notes that have not been obtained, it means that a record note is obtained at step S111. If all of the record notes in the electronic medical chart of the target patient have been obtained already, it means that no record note is obtained at step S111. When any record note was obtained, the process proceeds to step S113. When no record note was obtained, then the target-patient record analysis process is completed.

(Step S113) The term extraction unit 171 performs morphological analysis on the obtained record note, starting with its beginning. For example, the term extraction unit 171 performs the morphological analysis on each character string describing the contents of the record note, in order to extract and obtain a term from the character string, in order starting with the first one. In this connection, before performing the morphological analysis, the term extraction unit 171 may convert characters that are representable in both one byte and two bytes into characters in either one byte or two bytes in the record note. For example, before performing the morphological analysis, the term extraction unit 171 converts one-byte katakana into two-byte katakana, or two-byte alphabetical characters into one-byte alphabetical characters.

In addition, the term extraction unit 171 may eliminate two-byte numbers, one-byte numbers, and symbols from the record note. This is because such numbers and symbols are likely to cause errors in similarity calculation. However, many numbers and symbols included in the "physical record" type of record notes are meaningful. Therefore, for example, the term extraction unit 171 is designed not to eliminate numbers and symbols from the "physical record" type of record notes.

(Step S114) The term extraction unit 171 determines whether a new term was obtained through the morphological analysis. For example, in the case where the morphological analysis is done up to the last of the record note, and a process including lexical category check of all terms and so on is complete, no new term is obtained. If there is one or more terms that have not undergone the lexical category check or the like, a term may be obtained. If any term was obtained, the process proceeds to step S115. If no term was obtained, the process proceeds to step S120.

(Step S115) Since the term was obtained, the term extraction unit 171 checks the lexical category of the obtained term.

(Step S116) The term extraction unit 171 determines whether the lexical category of the obtained term needs to be registered. For example, lexical categories that need to be registered are defined in advance in the term extraction unit 171. The lexical categories to be registered include "noun," "verb," "adjective," and "adjectival verb," for example. "Particle" and "adverb" are likely to cause errors in similarity calculation, and therefore are excluded from registration. If the lexical category of the obtained term needs to be registered, the process proceeds to step S117. If the lexical category of the obtained term does not need to be registered, the process proceeds back to step S113.

(Step S117) The term extraction unit 171 checks whether the obtained term is included in the registration exclusion dictionary 121 stored in the registration exclusion dictionary storage unit 120. In this connection, in the case where a noun follows another noun, for example, the term extraction unit 171 recognizes a combination of these nouns as a different term. For example, in the case where "tendency" follows "improvement", three terms, "improvement," "tendency," and "improvement tendency", are taken as terms to be checked. In addition, in the case where a term includes four or more characters and the term ends with "-" (prolonged sound symbol), the term extraction unit 171 may delete "-." For example, this symbol is found when terms like "server" and "user" are written in Japanese.

(Step S118) The term extraction unit 171 determines whether to exclude the obtained term from registration. For example, if the obtained term is included in the registration exclusion dictionary 121, the term extraction unit 171 determines to exclude the term from registration. If the term is to be excluded from registration, the process proceeds back to step S113. If the term is not to be excluded from registration, the process proceeds to step S119.

(Step S119) The term extraction unit 171 adds the term obtained at step S113 to the term array of the record note obtained at step S111. Then, the process proceeds back to step S113.

(Step S120) Since no term was obtained at step S113, the term extraction unit 171 determines that the analysis of the obtained record note is complete, and then stores the term array of the record note obtained at step S111 in association with the record note in the target-patient-record-based term storage unit 130. Then, the process proceeds back to step S111.

As described above, the term array corresponding to each record note included in the electronic medical chart of the target patient is stored in the target-patient-record-based term storage unit 130.

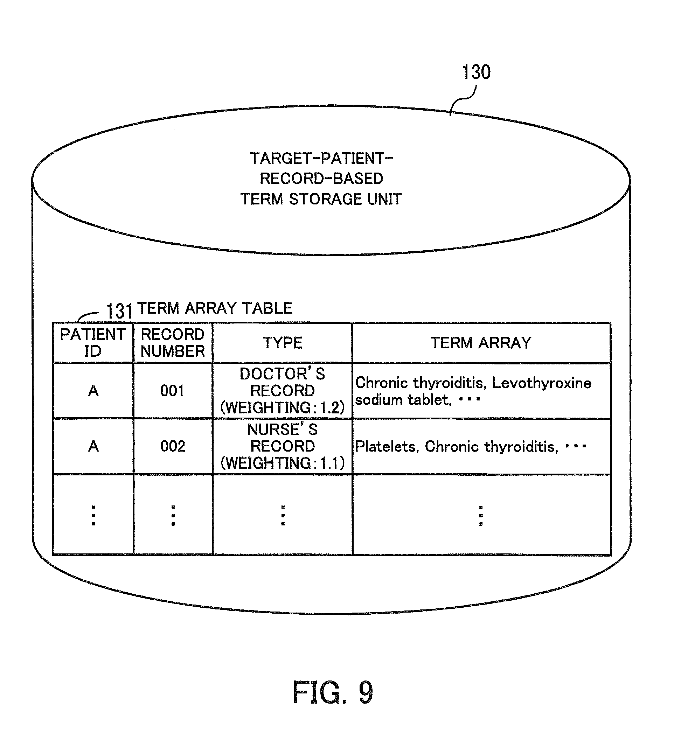

FIG. 9 illustrates an example of a data structure of a target-patient-record-based term storage unit. A term array table 131 is stored in the target-patient-record-based term storage unit 130. The term array table 131 is a data table that contains terms extracted from the record notes of the target patient, for each of the record notes. The term array table 131 includes fields for patient ID, record number, type, and term array. The patient ID field contains the patient ID of a target patient. The record number field contains the record number of a record note included in the electronic medical chart of the target patient. The type field indicates the type of the corresponding record note. The term array field lists the terms extracted from the corresponding record note.

The following describes an other-patient record analysis process.

FIG. 10 is a flowchart illustrating an example of how to perform an other-patient record analysis process. The other-patient record analysis process is performed after the target-patient record analysis process is completed.

(Step S131) The term extraction unit 171 obtains one unselected record note of the electronic medical chart of a patient (another patient) other than the target patient from the electronic medical chart storage unit 110. In this connection, the term extraction unit 171 may previously filter the record notes of the electronic medical chart of the other patient with SQL.

(Step S132) The term extraction unit 171 determines whether any record note was obtained at step S131. If the electronic medical chart of at least one of the other patients includes one or more record notes that have not been obtained, it means that a record note is obtained at step S131. If all of the record notes in the electronic medical charts of all the other patients, it means that no record note is obtained at step S131. If a record note was obtained, the process proceeds to step S133. If no record note was obtained, the other-patient record analysis process is completed.

(Step S133) The term extraction unit 171 performs the morphological analysis on the obtained record note, starting with its beginning, in order to obtain a term in order starting with the first one. This step is executed in the same way as step S113 of FIG. 8.

(Step S134) The term extraction unit 171 determines whether a new term was obtained through the morphological analysis. If a term was obtained, the process proceeds to step S135. If no term was obtained, the process proceeds to step S140.

(Step S135) Since the term was obtained, the term extraction unit 171 checks the lexical category of the obtained term.

(Step S136) The term extraction unit 171 determines whether to register the lexical category of the obtained term. If the lexical category of the obtained term needs to be registered, the process proceeds to step S137. If the lexical category of the obtained term does not need to be registered, the process proceeds back to step S133.

(Step S137) The term extraction unit 171 checks whether the obtained term is included in the registration exclusion dictionary 121 stored in the registration exclusion dictionary storage unit 120. This step is executed in the same way as step S117 of FIG. 8.

(Step S138) The term extraction unit 171 determines whether to exclude the obtained term from registration. If the term is to be excluded from registration, the process proceeds back to step S133. If the term is not to be excluded from registration, the process proceeds to step S139.

(Step S139) The term extraction unit 171 adds the term obtained at step S133 to the term array of the record note obtained at step S131. Then, the process proceeds back to step S133.

(Step S140) Since no term was obtained at step S133, the term extraction unit 171 determines that the analysis of the obtained record note is complete, and then calculates the degree of similarity (0 or greater and 1 or less) between the obtained record note and each of the plurality of record notes of the target patient. The term array extracted from each record note is used for the similarity calculation. For example, the term extraction unit 171 compares the term array generated at step S139 with the term array of the storage note stored in the target-patient-record-based storage unit 130 to calculate the degree of similarity with TF (Term Frequency)-IDF (Inverse Document Frequency). TF-IDF is a technique for calculating the degree of importance of a term in a document on the basis of the term occurrence frequency (TF) and inverse document frequency (IDF). Using the ID-IDF decreases the degree of importance of a term if it is included in many record notes and increases the degree of importance of a term if it occurs only in specific record notes.

For example, the term extraction unit 171 calculates the degree of similarity between each record note of the target patient and the obtained record note of the other patient using the degrees of importance of the terms extracted from the record notes in the vector space model. In the vector space model, vectors are created using the degrees of importance of the terms included in two record notes to be compared with each other, and the cosine of the angle θ (cosθ) between the two vectors is taken as the degree of similarity.

In addition, the term extraction unit 171 is able to define a weight for each type of record notes of electronic medical charts. In this case, a higher weight is defined for records of higher importance. For example, weights "1.2," "1.1," and "0.9" are defined for doctor's records, nurse's records, and other records, respectively. In addition, a weight for physical information may be set higher than that for the other record notes. A higher weight for the physical information increases the degrees of similarity of record notes of other patients who have similar physical features to the target patient. The term extraction unit 171 multiplies a degree of similarity calculated in the vector space model by the weight values corresponding to the types of the compared record notes. Then, the term extraction unit 171 takes the result of multiplication by the weights as a final degree of similarity.

(Step S141) The term extraction unit 171 determines whether the obtained record note has a high degree of similarity to any record note of the target patient. For example, the term extraction unit 171 arranges, with respect to each record note of the target patient, the record notes of the other patients in the descending order of the degree of similarity to the record note. If the obtained record note is one of the top 20 record notes, the term extraction unit 171 determines that the record note has a high degree of similarity. If the obtained record note has a high degree of similarity to any record note of the target patient, the process proceeds to step S142. If the obtained record note does not have a high degree of similarity to any record note of the target patient, the process proceeds back to S131.

In this connection, there is a case where, with respect to one record note of the target patient, a plurality of record notes of another patient falls in a predetermined number of top record notes in the similarity ranking. In this case, the term extraction unit 171 determines that the record notes other than the highest ranking one among the plurality of record notes of the other patient do not have high degrees of similarity.

(Step S142) The term extraction unit 171 stores the term array extracted from the obtained record note in association with one or a plurality of record notes of the target patient that have high degrees of similarity to the obtained record note in the similar-record-based term storage unit 140. In this connection, there is a case where, because of a new record note stored, the number of record notes of other patients associated with a record note of the target patient exceeds a predetermined value (for example, 20). In this case, the term extraction unit 171 deletes the term array of the record note with the lowest degree of similarity among the record notes of the other patients associated with the record note of the target patient from the similar-record-based term storage unit 140. Then, the process proceeds back to step S131.

As described above, the term arrays extracted from the record notes of other patients which are similar to each record note of the target patient are stored in the similar-record-based term storage unit 140.

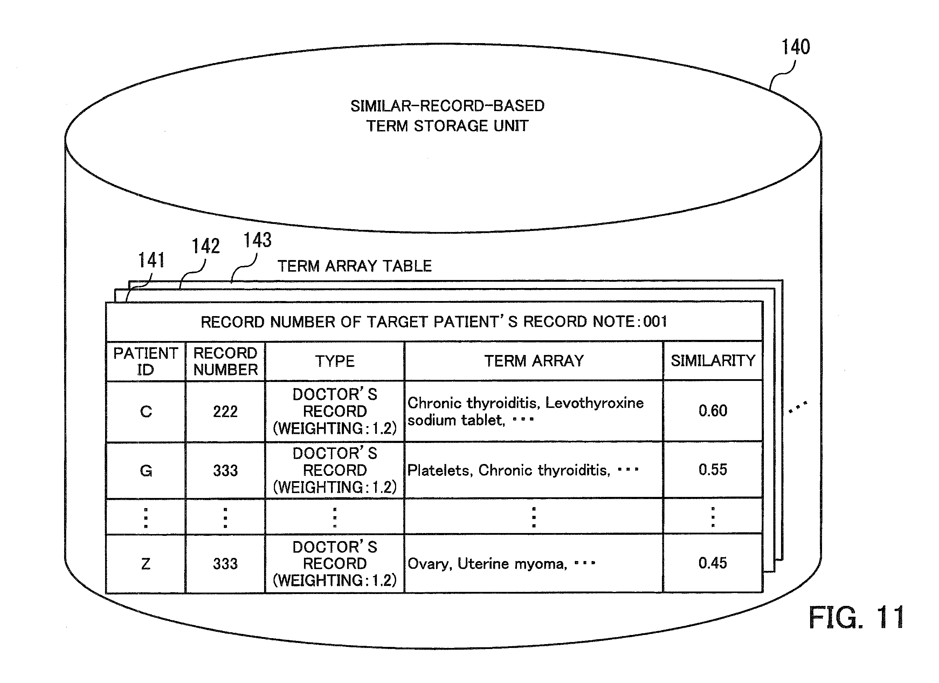

FIG. 11 illustrates an example of a data structure of a similar-record-based term storage unit. A term array table 141, 142, 143, ... for each record note of the target patient is stored in the similar-record-based term storage unit 140. Each term array table 141, 142, 143, ... is given the record number of a corresponding record note of the target patient. In addition, each term array table 141, 142, 143, ... includes fields for patient ID, record number, type, term array, and similarity.

The patient ID field contains the patient ID of another patient. The record number field contains the record number of the record note of the other patient which has similar contents to a record note of the target patient. The type field indicates the type of the record note. The term array field contains the term array extracted from the record note. The similarity field indicates the degree of similarity to the record note of the target patient.

As described above, each term array table 141, 142, 143, ... contains the term arrays of record notes of other patients which are similar to a record note of the target patient in association with the record note of the target patient. In this connection, the term arrays of a plurality of record notes of another patient are not registered in a single term array table. That is to say, the term array of only one record note per patient is allowed to be recorded in a single term array table. However, the term array of one record note of another patient may be registered in a plurality of term array tables.

A plurality of record notes of the target patient and the record notes of other patients which are similar to the record notes are associated with each other by the term array tables 141, 142, 143, ... stored in the similar-record-based term storage unit 140.

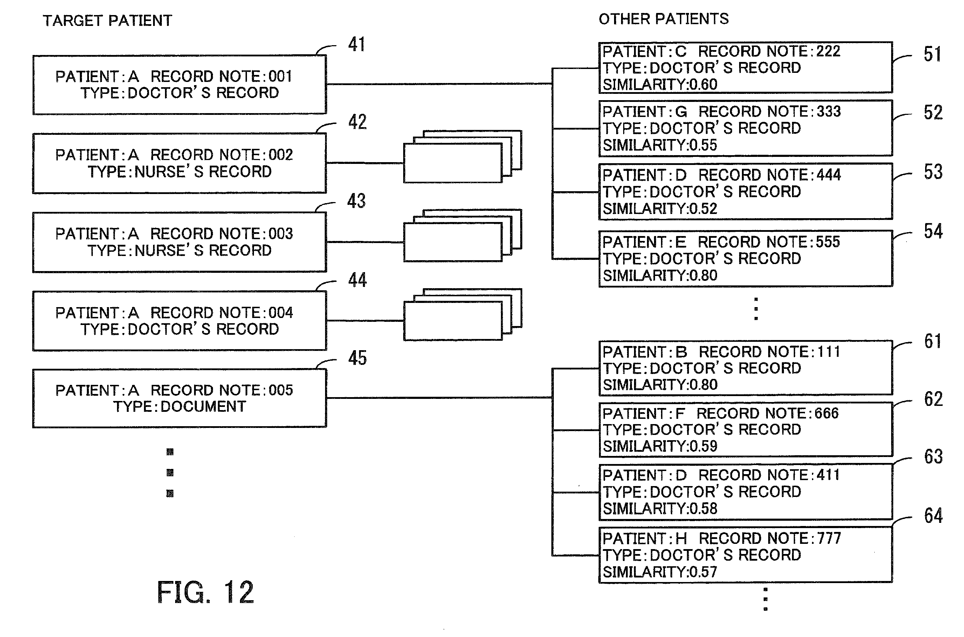

FIG. 12 illustrates similarity relationships between record notes. A predetermined number of top record notes of other patients in a similarity ranking are associated with each of the record notes 41, 42, 43, 44, 45, ... of the target patient. For example, a predetermined number of record notes 51, 52, 53, 54, ... of other patients which are similar to the record note 41 of the target patient are associated with the record note 41. A predetermined number of record notes 61, 62, 63, 64, ... of other patients which are similar to the record note 45 of the target patient are associated with the record note 45. If the target patient has 1000 record notes of and the top 20 record notes of other patients in the similarity ranking are associated with each of the record notes of the target patient, term arrays are extracted from 20000 record notes of the other patients.

Patients (similar patients) having similar treatment histories to the target patient are determined based on the record notes of the other patients associated with each of the plurality of record notes of the target patient.

FIG. 13 is a flowchart illustrating how to perform a similar patient determination process. This process is performed after the other-patient record analysis process is completed.

(Step S151) The similar patient determination unit 172 calculates, for each patient ID of the other patients, the total degree of similarity of the record notes of the other patient which are similar to any of the record notes of the target patient. For example, the similar patient determination unit 172 obtains, for each patient ID of the other patients, the degrees of similarity from the records having the patient ID from the similar-record-based term storage unit 140. Then, the similar patient determination unit 172 calculates the total degree of similarity for each patient ID.

(Step S152) The similar patient determination unit 172 compares the total degrees of similarity with each other, and determines a predetermined number (for example, 20) of top patients as similar patients to the target patient. For example, the similar patient determination unit 172 sorts the patient IDs of the other patients in descending order of the total degree of similarity. The similar patient determination unit 172 extracts a predetermined number of top patient IDs on the basis of the sorting result. Then, the similar patient determination unit 172 stores the extracted patient IDs in the similar patient information storage unit 150.

(Step S153) The similar patient determination unit 172 obtains the term arrays of the record notes of the similar patients. For example, the similar patient determination unit 172 extracts records with the patient IDs of the similar patients from the similar-record-based term storage unit 140, and eliminates the others. The similar patient determination unit 172 then stores the extracted records in the similar-patient-record-based term storage unit 160.

As described above, similar patients are specified. With this process, for example, "patients who have many high-ranking record notes in similarity to the record notes of the electronic medical chart of the target patient" are specified as the similar patients. In addition, "patients who do not have many record notes which are similar to record notes of the electronic medical chart of the target patient but have some record notes that each have high degrees of similarity" may be specified as the similar patients.

A list of the specified similar patients is stored in the similar patient information storage unit 150.

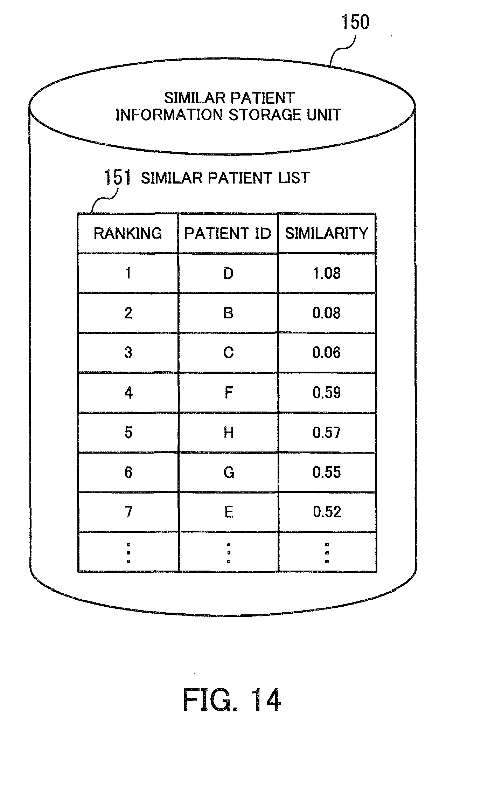

FIG. 14 illustrates an example of a data structure of a similar patient information storage unit. A similar patient list 151 is stored in the similar patient information storage unit 150. The similar patient list 151 lists top patients in a similarity raking. Referring to the example of FIG. 14, the similar patient list 151 indicates the positions of patients in the ranking, the patient IDs, and the degrees of similarity.

With regard to the patients registered in the similar patient list 151, the term arrays extracted from record notes of the patients are stored in the similar-patient-record-based term storage unit 160.

FIG. 15 illustrates an example of a data structure of a similar-patient-record-based term storage unit. A term array table 161, 162, 163, ... for each record note of the target patient is stored in the similar-patient-record-based term storage unit 160. The term array tables 161, 162, 163, ... have the same data structure of the term array tables 141, 142, 143, ... stored in the similar-record-based term storage unit 140 illustrated in FIG. 11. However, only records containing the patient IDs of similar patients are registered in the term array tables 161, 162, 163, ... in the similar-patient-record-based term storage unit 160. For example, a record containing a patient ID "Z" is included in the term array table 141 in the similar-record-based term storage unit 140 illustrated in FIG. 11. It is now assumed that the patient with the patient ID "Z" is not a similar patient. In this case, the record with the patient ID "Z" is not included in the term array table 161 in the similar-patient-record-based term storage unit 160 illustrated in FIG. 15.

Terms that are useful for the medical care of the target patient are extracted from the terms stored in the similar-patient-record-based term storage unit 160.

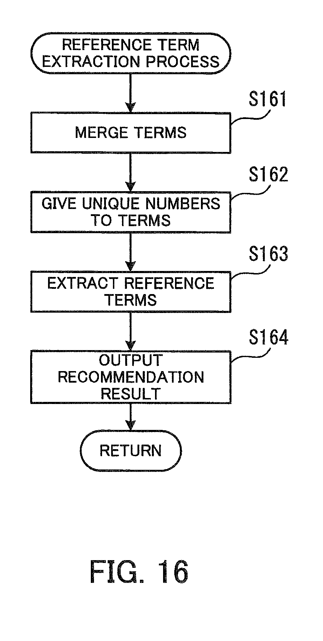

FIG. 16 is a flowchart illustrating how to perform a reference term extraction process. This process is performed after the similar patient determination process is completed.

(Step S161) The reference term extraction unit 173 combines (merges) the terms extracted from the record notes of the target patient and the terms extracted from the record notes of the similar patients.

(Step S162) The reference term extraction unit 173 gives a unique number (term ID) to each term after the merging. For example, the reference term extraction unit 173 uses the position number of a term in the order of occurrence of terms in the merged term array as the term ID of the term. At this time, the associations between terms and record notes are cleared and a list of occurring terms is generated for each patient.

(Step S163) The reference term extraction unit 173 extracts reference terms from the occurring term list of each patient. For example, the reference term extraction unit 173 selects reference terms by applying the collaborative filtering (CF) technique. In the collaborative filtering, for example, users' actions are recorded, and other users having similar actions to a specified user are considered as users having similar preferences to the specified user. Then, it is expected that the specified user likes similar actions to the other users having the similar preferences, and actions that were not taken by the specified user but were taken by the other users having the similar preferences are recommended to the specified user. By using the "terms" extracted from record notes in place of the "actions" used in the collaborative filtering, it is possible to extract terms that have not been considered for the target patient but are considered useful for future medical care from the record notes of the other users who have similar details of medical care to the target patient.

Therefore, the reference term extraction unit 173 extracts terms (in plurality) that are not included in the occurring term list of the target patient from the occurring term lists of the other patients determined to have similar preferences with the collaborative filtering, as reference terms.

(Step S164) The reference term extraction unit 173 outputs the extracted reference terms as a recommendation result. For example, the reference term extraction unit 173 transmits the list of reference terms to the terminal device having made the recommendation request.

As described above, it is possible to extract terms useful for the medical care of the target patient from the terms included in the record notes of other patients having similar details of medical care.

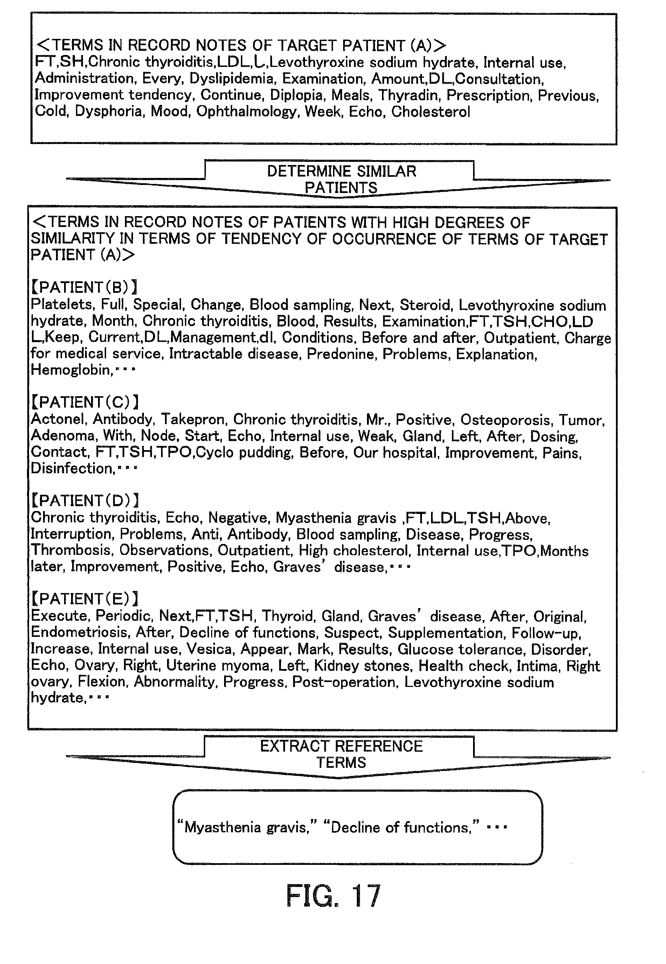

FIG. 17 illustrates an example of recommendation. Referring to the example of FIG. 17, the patient with a patient ID "A" is taken as a target patient. In this case, similar patients are determined by comparing terms included in the record notes of the patient with the patient ID "A" and the other patients. Referring to the example of FIG. 17, the patients with patient IDs "B," "C," "D," "E" are determined as similar patients.

Then, reference terms are extracted by comparing the terms included in the record notes of the target patient (patient ID "A") with the terms included in the record notes of the similar patients (patient IDs "B," "C," "D," and "E"). For example, as tendencies of the patients with high degrees of similarity to the patient with the patient ID "A," many terms related to thyroiditis occur, and "Levothyroxine sodium hydrate" occurs many times as a medical agent. As a tendency of the patient with patient ID "A," the terms "myasthenia gravis" and "decline of functions" do not occur. Therefore, the terms "myasthenia gravis" and "decline of functions" are extracted as reference terms and are sent as a recommendation result.

As described above, it is possible to detect similar patients corresponding to electronic medical charts whose contents are similar to those of the electronic medical chart of the target patient, extract terms (keywords) useful for the medical care of the target patient from the electronic medical charts of the similar patients, and propose them, for example, to a target patient's doctor. As a result, it is possible to prevent overlooking any important matters to be considered for the symptoms of the patient when a treatment plan is determined, to thereby appropriately determine a treatment plan for the target patient and improve the quality of medical care. In addition, it is possible to reduce the degree of dependency on the abilities of individual doctors when treatment plans are determined and to thereby provide a high and uniform level of medical service.

In addition, the degree of similarity between each of the plurality of record notes included in the medical record of a target patient and each of the plurality of record notes included in the medical record of each of the other patients is calculated, the total degree of similarity is calculated for each of the other patients, and a predetermined number of other patients are taken in descending order of the total degree of similarity as similar patients. This enables the target patient's doctor to determine an appropriate treatment plan with reference to the details of medical care for a large number of similar patients. In particular, since a large-scale hospital has a massive number of electronic medical charts, a treatment plan for the target patient is determined with reference to the past medical data of various patients, which makes it possible to provide more appropriate medical care.

Still further, a predetermined number of record notes of other patients are extracted in descending order of the degree of similarity with respect to each of the plurality of record notes included in the medical record of the target patient, and the total degree of similarity of the extracted record notes is calculated for each of the other patients. That is, unuseful record notes are eliminated during the extraction of record notes. As a result, even if a patient who suffers from similar symptoms to the target patient has an electronic medical chart including a large number of record notes that are not useful for the target patient due to treatments for different symptoms, the patient who suffers from the similar symptoms is appropriately detected. For example, consider the case where the target patient has treatments for diabetes. In this case, other patients with diabetes are expected to be extracted as similar patients. Now, also assume that a patient with diabetes has a long-term dermatology treatment, irrespective of diabetes. If electronic medical charts as a whole are compared with each other in this situation, a possibility of the occurrence of diabetes-related terms regarding the patient with diabetes decreases, and therefore the patient with diabetes may not be determined as a similar patient. By contrast, in the second embodiment, record notes are compared with each other for similarity relationship and record notes with low degrees of similarity are eliminated, so that the record notes relating to treatments other than diabetes treatment are eliminated, and the patient with diabetes is appropriately determined as a similar patient. That is, the similar patient determination has an improved accuracy.

Still further, weighting based on the types of record notes may be performed in calculating the degree of similarity between record notes. For example, a result of similarity calculation based on the contents of two record notes, which are objects for calculating the degree of similarity, is multiplied by the weights corresponding to the two records notes, and the multiplication result is taken as the degree of similarity between the two record notes. For example, a weight for record notes indicating doctors' medical care may be defined higher than that for record notes indicating nurses' care. This enables a doctor to refer to the details of other doctors' medical care when determining a treatment plan. In the case where a nurse makes a recommendation request for appropriate nursing care for symptoms of a patient, a weight for record notes indicating nurses' care is defined higher than that for record notes indicating doctors' medical care, thereby allowing the nurse to receive an appropriate recommendation result.

Still further, physical information is included in record notes. This makes it possible to output, as a recommendation result, terms indicating treatments that were given to other patients who were similar in age, sex, weight, height, or the like to a target patient and have similar details of medical care to the target patient, and that have not been given to the target patient.

Still further, a weight for record notes including physical information may be defined higher than that for the other types of record notes. By defining the highest weight for physical information, it becomes possible to preferentially determine other patients having similar physical features to the target patient as similar patients. In the recommendation using such weighting, terms useful for determining a treatment plan for a disease whose symptoms and treatment details vary depending on, for example, sex and age, are obtained as a recommendation result.

Still further, by extracting the terms which do not occur in the medical record of the target patient from the terms occurring in the plurality of record notes included in the medical records of similar patients, it becomes possible to prevent unuseful terms from being extracted. As a result, a doctor who receives a recommendation result is able to promptly determine a treatment plan with reference to the recommendation result without being misled by unuseful terms.

The foregoing is considered as illustrative only of the principles of the present invention. Further, numerous modifications and changes will readily occur to those skilled in the art, it is not desired to limit the invention to the exact construction and applications shown and described, and all suitable modifications and equivalents may be regarded as falling within the scope of the invention in the appended claims and their equivalents.

### Reference Signs List

- 1: Storage means
- 1a, 1b, 1c,: Medical records
- 1a-1, 1a-2, 1a-3, 1b-1, 1b-2, 1b-3,: Record notes
- 2: Specifying means
- 3: Similar patient information
- 4: Extraction means
- 5, 6: Term arrays
- 7: Reference terms
- S: Medical information analysis apparatus

## Claims

1. A medical information analysis program that causes a computer to perform a process comprising:
specifying a second patient whose medical record is similar to a medical record of a first patient with reference to medical records of a plurality of patients; and
extracting information from the medical record of the second patient.

2. The medical information analysis program according to claim 1, wherein the specifying a second patient includes calculating a degree of similarity between each of a plurality of record notes included in the medial record of the first patient and each of a plurality of record notes included in medical records of other patients, and calculating a total degree of similarity between record notes for each of the other patients.

3. The medical information analysis program according to claim 2, wherein the specifying a second patient includes extracting a predetermined number of record notes of the other patients in descending order of the degree of similarity, with respect to each of the plurality of record notes of the medical record of the first patient, and calculating the total degree of similarity of the extracted record notes for each of the other patients.

4. The medical information analysis program according to claim 2 or 3, wherein the calculating a degree of similarity between record notes includes performing weighting based on types of record notes to multiply a result of similarity calculation based on contents of two record notes, which are objects for calculating the degree of similarity, by weights corresponding to the two record notes, and taking a result of multiplication as the degree of similarity between the two record notes.

5. The medical information analysis program according to any one of claims 1 to 4, wherein the medical records of the plurality of patients include physical information indicating physical features of the patients.

6. The medical information analysis program according to any one of claims 1 to 5, wherein the specifying a second patient includes calculating a degree of similarity between the medical record of the first patient and the medical record of each of other patients, and taking a predetermined number of other patients in descending order of the degree of similarity as second patients.

7. The medical information analysis program according to any one of claims 1 to 6, wherein extracting of a term includes extracting a term which does not occur in the medical record of the first patient from terms occurring in the medical record of the second patient.

8. A medical information analysis apparatus comprising:
specifying means for specifying a second patient whose medical record is similar to a medical record of a first patient with reference to medical records of a plurality of patients; and
extraction means for extracting information from the medical record of the second patient.

9. A medical information analysis method comprising:
specifying, by a computer, a second patient whose medical record is similar to a medical record of a first patient with reference to medical records of a plurality of patients; and
extracting, by the computer, information from the medical record of the second patient.
